# EUROPÄISCHE PATENTSCHRIFT

(11) **EP 4 107 123 B1**
(45) Veröffentlichungstag und Bekanntmachung des Hinweises auf die Patenterteilung: **17.07.2024**
(21) Anmeldenummer: 20706438.7
(22) Anmeldetag: 18.02.2020
(51) Int. Cl.: C01B 39/02, C07C 5/31, C07C 13/21, B01J 29/70

(54) **VERFAHREN ZUR HERSTELLUNG VON LIMONEN UND ZUSAMMENSETZUNG ENTHALTEND LIMONEN**
METHOD FOR PRODUCING LIMONENE AND COMPOSITION CONTAINING LIMONENE
PROCÉDÉ DE PRÉPARATION DE LIMONÈNE ET COMPOSITION CONTENANT DU LIMONÈNE

(43) Veröffentlichungstag der Anmeldung: 28.12.2022
(73) Patentinhaber: Symrise AG, 37603 Holzminden Niedersachsen (DE)
(72) Erfinder: BUGDAHN, Nikolas, 37603 Holzminden (DE); JAIME, Diego, 41540 Dormagen (DE); RUSSBÜLDT, Bernhard, 37671 Höxter (DE); STRÜVER, Frank, 31789 Hameln (DE)
(74) Vertreter: Fabry, Bernd
(86) Internationale Anmeldenummer: PCT/EP2020/054230
(87) Internationale Veröffentlichungsnummer: WO 2021/164850

(56) Entgegenhaltungen:
- WO-A1-2011/061204
- MA XUETAO ET AL: "Highly selective isomerization of biomass [beta]-pinene over hierarchically acidic MCM-22 catalyst", MICROPOROUS AND MESOPOROUS MATERIALS, ELSEVIER, AMSTERDAM, NL, vol. 237, 23 September 2016 (2016-09-23), pages 180 - 188, XP029777585, ISSN: 1387-1811, DOI: 10.1016/J.MICROMESO.2016.09.040

## Beschreibung

### GEBIET DER ERFINDUNG

Die Erfindung befindet sich auf dem Gebiet der Terpenverbindungen und betrifft ein Verfahren zur katalytischen Umlagerung von beta-Pinen in Limonen.

### TECHNOLOGISCHER HINTERGRUND

Limonen ist ein Naturstoff aus der Gruppe der monozyklischen Terpene, der in zwei Enantiomeren vorkommt 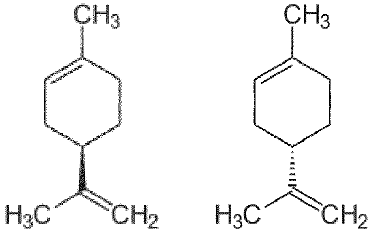 dem (*R*)-(+)-Limonen (auch als D-(+)-Limonen oder kurz (+)-Limonen bezeichnet) und dem (*S*)-(-)-Limonen [auch als L-(-)-Limonen oder kurz (-)-Limonen bezeichnet]. Das Racemat der beiden Enantiomeren wird auch Dipenten genannt.

Limonen ist das in Pflanzen am häufigsten vorkommende Monoterpen. (*R*)-(+)-Limonen ist vor allem in Pomeranzenschalenöl, in Kümmelöl, in Dillöl, in Korianderöl, in Zitronenöl (ca. 65 %) und in Orangenöl (meist >90 %) enthalten. Es weist einen orangenartigen Geruch auf. Dagegen ist (*S*)-(-)-Limonen in Edeltannen- und in Pfefferminzöl enthalten und riecht nach Terpentin. Das racemische Limonen kommt unter anderem im Kienöl, im sibirischen Fichtennadelöl, Neroliöl, Muskatnussöl und Campheröl vor.

Die Biosynthese von Limonen geht von Geranylpyrophosphat (GPP) aus.

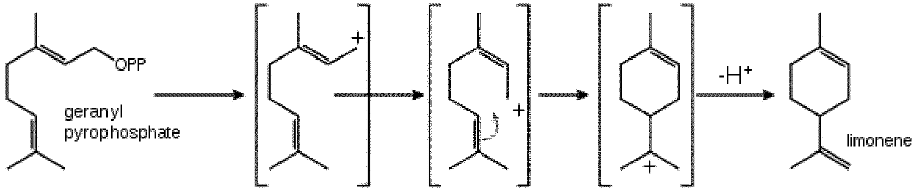

Limonen ist ein essentieller Rohstoff für die Riechstoffindustrie und Ausgangsmaterial für eine Vielzahl von Anwendungen und Produkten. Insbesondere wird es in letzter Zeit auch als "grünes" Lösungsmittel verwendet und stellt eine Alternative zu den aus ökologischer Sicht bedenklichen BTX-Lösungsmitteln dar.

Beta-Pinen stellt zwar eine gut verfügbare Quelle für die Herstellung von Limonen dar, die Schwierigkeit bei der sauer katalysierten Isomerisierung von β-Pinen besteht jedoch in der Möglichkeit der Bildung eines breiten Produktspektrums von bi-, tri- und monocyclischen Terpenen über eine Reihe von Gleichgewichtsreaktionen, die vermieden werden muss, um eine hohe Limonenselektivität zu erreichen. Ein Ausschnitt aus diesem möglichen Produktspektrum ist nachfolgend dargestellt.

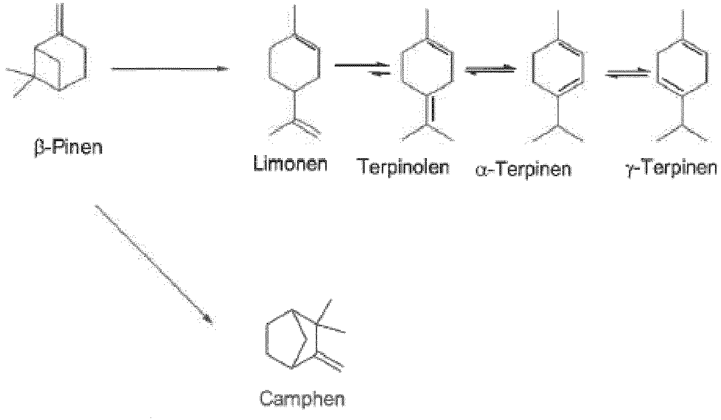

### RELEVANTER STAND DER TECHNIK

Zu den ersten einschlägigen Veröffentlichungen gehören die beiden Schutzrechte US 3,780,124 (DAVIS) und US 3,780,125 (TAKACS) aus dem Jahre 1973, in denen vorgeschlagen wird, Zeolithe für die Umlagerung von alpha-Pinen mit lod zu beaufschlagen.

Aus der US 4,508,930 (WIDEMANN) ist die Umlagerung von Terpenen in Limonen in Gegenwart von Alkalisulfid-Trägerkatalysatoren bei hohen Temperaturen bekannt.

Die Isomerisierung von Pinen zu Limonen und weiteren Nebenprodukten in Gegenwart von Zeolithen ist bereits aus der US 3,270,075 (GLIDDEN) bekannt Die Reaktion findet in der Flüssigphase bei 65 bis 110 °C statt. Der Katalysator wird allgemein beschrieben als Meₓ/n[(AlO₂)x(SiO₂)_{y]} * z H₂O und konkret genannt wird:

Na₈₆[(AlO₂)₈₆(SiO₂)₁₀₆] * 267 H₂O.

WO 2011 061204 A1 betrifft einen heterogenen Katalysator mit einem Gehalt von 1 bis 9 Gew.-% PSH3 zur Isomerisierung von Alkylaromaten in der Gasphase. Beta-Pinen fällt jedoch nicht unter die Definition eines Alkylaromaten.

Die beiden Chinesischen Patente CN 102126904 B **und** CN 102343277 B der Universität Dalian beschreiben ebenfalls die Isomerisierung von Pinenen zu Limonen in Gegenwart heterogener saurer Katalysatoren. Dabei kommen als Katalysatoren saure Molekularsiebe zum Einsatz, die zum einen mit Halogeniden und zum anderen mit Basen nachbehandelt worden sind.

Ferner sei auf die beiden Aufsätze von Ma et.al. in MICROPOROUS AND MESO-POROUS MATERIAL 237, S. 180-188 (2017**)** sowie Golets et al. in CHEM. REV. 115, S. 3141-3196 (2017**)** verwiesen. Ersterer betrifft die Isomerisierung von beta-Pinen in Gegenwart von MCM-22 Katalysatoren, bei der eine Mischung aus Limonen und Campher anfällt. Die Umsetzung erreicht dabei nach 3 h etwa 100 %, die Selektivität zu Limonen liegt bei mindestens 80 %. Die Verwendung von Zeolithen des Typs PSH-3 wird jedoch nicht offenbart.

Nachteilig ist, dass die aus dem Stand der Technik bekannten Verfahren weder hinsichtlich der Ausbeuten noch der Selektivitäten ökonomisch sinnvolle Alternativen darstellen. Die Verfahren arbeiten zudem in der Regel bei sehr hohen Temperaturen, erfordern die Mitverwendung toxischer Lösungsmittel, lassen sich nicht kontinuierlich durchführen und weisen bezüglich der Katalysatoren zu geringe Standzeiten auf. Zudem enthalten die Reaktionsprodukte häufig unerwünschte Nebenprodukte wie insbesondere Terpinene in Mengen, die die Produktqualität negativ beeinflussen.

### AUFGBE DER ERFINDUNG

Aufgrund deutlich gestiegener Preise für limonenhaltige Rohstoffe besteht ein Bedarf, Limonen aus deutlich günstigeren und in größeren Mengen zur Verfügung stehen Edukten, speziell beta-Pinen in einfacher und ökonomisch sinnvoller Weise herzustellen.

Eine erste Aufgabe der vorliegenden Erfindung hat daher darin bestanden, ein Verfahren zur Oxidation von beta-Pinen zu Limonen zur Verfügung zu stellen, welches Ausbeuten und Selektivitäten von mindestens 75 % aufweist und dabei als Nebenprodukt weitestgehend (d.h. etwa 15 bis 20 Gew.-%) nur Camphen liefert. Das Limonen sollte dabei spezifisch (> 90 Gew.-%) als L-Isomer anfallen.

Eine weitere Aufgabe hat darin bestanden, das Verfahren so zu führen, dass auf die Mitverwendung von toxischen Lösungsmitteln, speziell aus der BTX-Reihe verzichtet werden kann. Die Reaktion sollte unter 100 °C auch kontinuierlich durchführbar sein und der Katalysator eine so hohe Standzeit besitzen, dass er mehrfach auch ohne Aufarbeitung eingesetzt und wiederverwertet werden kann.

### BESCHREIBUNG DER ERFINDUNG

Ein erster Gegenstand der Erfindung betrifft ein Verfahren zur Herstellung von Limonen, umfassend oder bestehend aus den folgenden Schritten:
(a) Bereitstellen von beta-Pinen oder einem beta-Pinen enthaltenden Ausgangsstoff;
(b) Versetzen des Ausgangsstoffes mit einer katalytisch wirksamen Menge eines MWW-Zeolithen vom Typ PSH-3;
(c) Erhitzen der Reaktionsmischung auf eine Temperatur im Bereich von 60 bis 100 °C und gegebenenfalls
(d) Abtrennen des Limonens oder einer an Limonen angereicherten Fraktion aus dem Sumpf.

Die Umlagerung folgt dabei dem folgenden Reaktionsschema:

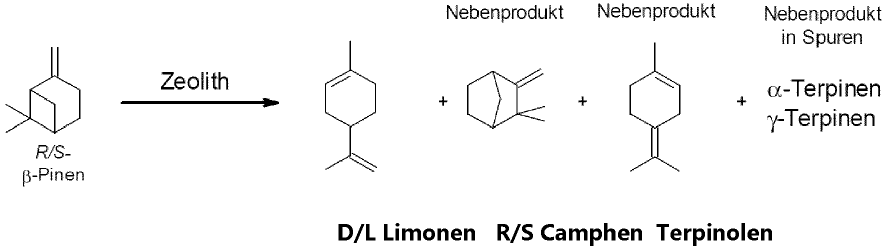

Überraschenderweise wurde gefunden, dass aus der großen Gruppe der für diese Reaktion grundsätzlich bekannten Zeolithe gerade solche vom MWW-Typ PSH-3 das oben geforderte Anforderungsprofil in allen Einzelheiten erfüllen. Die Umlagerung kann bei Temperaturen von typisch 70 bis 85 °C batchweise oder kontinuierlich durchgeführt werden, wobei als Lösungsmittel ökologisch unbedenkliche Essigsäureester zum Einsatz gelangen. Die Katalysatoren weisen eine hohe Standzeit auf und lassen sich ohne Aufarbeitung vielfach wiederverwenden und liefern als Reaktionsprodukt ein fast enantiomerenreines L-Limonen mit Ausbeuten und Selektivitäten von mehr als 75 %, vorzugsweise von 85 bis 100 %, insbesondere von 90 bis 99 % und ganz besonders bevorzugt von 95 bis 98 %, wobei als Nebenprodukt fast ausschließlich nur Camphen gebildet wird.

Die Erfindung schließt die Erkenntnis ein, dass die Selektivität hin zum L-Isomer mit der Reinheit des beta-Pinens korreliert.

Es sei darauf hingewiesen, dass beta-Pinen in zwei enantiomeren Formen, nämlich (S,S) und (R,R) vorliegen kann. Dementsprechend werden mit dem Begriff "beta-Pinen" im Sinne der vorliegenden Erfindungen beide Formen oder deren Gemische umfasst. Das bevorzugte Einsatzmaterial ist das (1S, 5S) beta-Pinen, das auch als (-)-beta-Pinen bezeichnet wird. Besonders bevorzugt ist indes das (1R, 5R) beta-Pinen, das auch als (+)-beta-Pinen bezeichnet wird, da sich auf diese Weise enantiomerenreines D-Limonen erhalten lässt.

### Katalysatoren

Unter der Bezeichnung "MWW" versteht man einen Zeolith-Typ, wie er in der nachfolgenden Abbildung wiedergegeben wird (vgl. *pubs.acs.org*/*doi*/*abs*/*10.1021*/*jp972319k):*

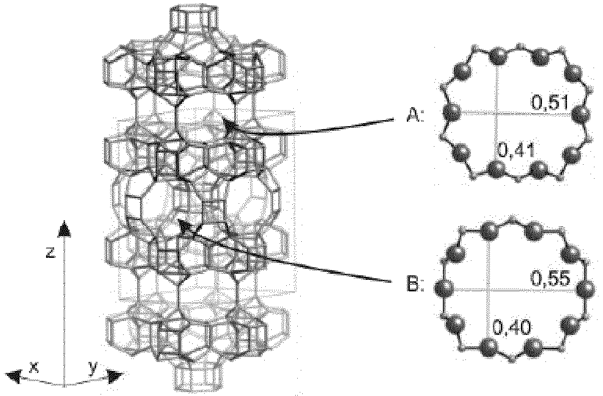

MWW-Zeolithe bestehen aus zwei voneinander getrennten Zehnringporensystemen. Dabei werden Varianten A und B unterschieden, bei denen die Hohlräume einmal linear und zum anderen sinusförmig verknüpft sind.

Erfindungsgemäß eingesetzt werden Zeolithen vom Typ PSH-3, die der Formel

M_{2/n}O*Al₂O₃*(20-150)SiO₂

entsprechen und beispielsweise aus der EP 0064205 B1 (BAYER) bekannt sind. Es ist bevorzugt, die PSH-3 Zeolithe vor dem Einsatz zu kalzinieren und/oder durch Säurebehandlung zu aktivieren. Die Kalzinierung kann dabei vorzugsweise über einen Zeitraum von etwa 1 bis etwa 10 Stunden und insbesondere 4 bis 6 Stunden bei einer Temperatur von etwa 400 bis etwa 1.000 °C und vorzugsweise 400 bis 600 °C erfolgen. Für die Säureaktivierung eignet sich besonders Salpetersäure.

Als wirksame Katalysatormenge kann eine Menge von etwa 1 bis etwa 5 Gew.-% und insbesondere etwa 2 bis 4 Gew.-% Zeolith bezogen auf die Menge an beta-Pinen angesehen werden.

### Umlagerungsreaktion

Die Reaktion wird vorzugsweise in Gegenwart von Lösungsmitteln durchgeführt. Hierfür eignen sich insbesondere Ester der Essigsäure mit einem aliphatischen C1-C4 Alkohol, vorzugsweise wird Ethylacetat eingesetzt,

Üblicherweise setzt man das beta-Pinen und das Lösungsmittel im Gewichtsverhältnis von etwa 10:1 bis 1:10 ein. Besonders bevorzugt ist ein Gewichtsverhältnis von etwa 2:1 bis 5:1

Die Reaktion wird vorzugsweise bei Temperaturen unter 150 °C. vorzugsweise unter 125 °C und besonders bevorzugt unter 100 °, nämlich typisch bei etwa 70 bis etwa 75 °C durchgeführt. Typische Reaktionszeiten liegen bei etwa 1 bis etwa 10 Stunden und insbesondere etwa 2 bis etwa 5 Stunden.

Die Umlagerungsreaktion kann batchweise, insbesondere aber auch kontinuierlich durchgeführt werden. Der Katalysator weist hohe Standzeiten auf und kann daher nach der Reaktion gegebenenfalls ohne vorherige Aufarbeitung wiederverwendet werden. Dazu reicht es bei der Batch-Fahrweise aus, den Katalysator im Sumpf zu belassen und einfach frisches Edukt beizufügen. Auf diese Weise lassen sich 10 bis 15 Zyklen fahren, ohne dass es zu signifikanten Rückgängen in Umsatz und Selektivität kommt. Die Standzeit des Katalysators im kontinuierlichen Betrieb liegt bei einigen Wochen. Optional kann der Reaktionsmischung eine hochsiedende Verbindung wie beispielsweise Polyole, Polyether, Polyester oder Silikonöle zugesetzt werden.

### GEWERBLICHE ANWENDBARKEIT

Ein letzter Gegenstand der vorliegenden Erfindung betrifft die Verwendung eines Zeolithen vom PSH-3 Typ, gegebenenfalls nach vorheriger Kalzinierung und/oder Säureaktivierung, zur Umlagerung von beta-Pinen in Limonen, vorzugsweise in einem Verfahren wie oben erläutert.

### BEISPIELE

### BEISPIEL 1

### Umlagerung von beta-Pinen zu Limonen mit frischem Katalysator

In einem 500 ml-Dreihalskolben mit Rührer, Rückflusskühler und Destillationsbrücke wurden 100 g (0,734) einer Handelsware beta-Pinen vorgelegt und mit 25 g Ethylacetat versetzt. Anschließend wurden 2 g (entsprechend 2 Gew.-% bezogen auf beta-Pinen) Zeolith-PSH-3 (bei 550 °C über 5 Stunde kalziniert) hinzugegeben. Die Mischung wurde unter Rühren auf 70 °C erhitzt und Proben nach 1,5 und 2 Stunden gezogen und analysiert. Nach einer Reaktionszeit von 2 h wurde das Reaktionsgemisch über Kopf destilliert und ebenfalls gaschromatographisch analysiert. Die Ergebnisse sind in **Tabelle 1** wiedergegeben:

**Tabelle 1**

| Zusammensetzung Sumpf und Destillat (GC-%) | | | |
|---|---|---|---|
| **Komponenten** | **Sumpf (nach 1,5 h)** | **Sumpf (nach 2 h)** | **Destillat (nach 2 h)** |
| α-Pinen | 1.65 | 1,26 | 0,98 |
| Camphen | 17,57 | 17,17 | 16,86 |
| ▪ -Pinen | 0,09 | 0,15 | 0,22 |
| Limonen* | 761,05 | 70,26 | 73,83 |
| α-Terpinen | 1,18 | 1,50 | 1,75 |
| γ-Terpinen | 1,00 | 1,07 | 1,11 |
| Terpinolen | 2,96 | 70,37 | 3,16 |
| ***Selektivität* (%)** | ***71,11*** | ***70,37*** | ***73,99*** |

| | | | |
|---|---|---|---|
| *) > 90 % L-Limonen | | | |

### BEISPIEL 2

### Umlagerung von beta-Pinen zu Limonen mit wiederverwertetem Katalysator

Beispiel 1 wurde wiederholt, jedoch der Katalysator nach dem Abtrennen des Destillats insgesamt 9 weitere Male mit frischem Edukt versetzt und die Reaktion im 10. Zyklus bei 85 °C durchgeführt. Die Zusammensetzung des Sumpfes des 10. Zyklus wurde nach 5, 8, 9 und 10 Stunden Reaktionszeit analysiert, ebenso die Zusammensetzung des nach dem 10. Durchgang erhaltenen Destillats. Die Ergebnisse sind in **Tabelle 2** zusammengefasst:

**Tabelle 2**

| Zusammensetzung Sumpf und Destillat (GC-%) | | | | | |
|---|---|---|---|---|---|
| **Komponenten** | **Sumpf (nach 5 h)** | **Sumpf (nach 8 h)** | **Sumpf (nach 9 h)** | **Sumpf (nach 10 h)** | **Destillat (nach 10 h)** |
| α-Pinen | 1,11 | 1,23 | 1,32 | 1,36 | 1.51 |
| Camphen | 12,92 | 14,56 | 15,31 | 15,73 | 17,64 |
| ▪-Pinen | 17,45 | 4,36 | 2,55 | 1,40 | 1,32 |
| Limonen* | 58,65 | 68,80 | 69,72 | 71,09 | 77,31 |
| α-Terpinen | 0,15 | 0,20 | 0,22 | 0,28 | 0,26 |
| γ-Terpinen | 0,19 | 0,24 | 0,25 | 0,26 | 0,27 |
| Terpinolen | 0,39 | 0,50 | 0,52 | 0,55 | 0,54 |
| ***Selektivität (%)*** | ***71,05*** | ***71,94*** | ***71,54*** | ***72,10*** | ***78,34*** |

| | | | | | |
|---|---|---|---|---|---|
| *) > 90 % L-Limonen | | | | | |

### BEISPIEL 3

### Kontinuierliches Verfahren

Aus einem Vorratsbehälter mit beta-Limonen/Essigsäureethylester (Gewichtsverhältnis 4:1) wurde mit Hilfe einer HPLC-Pumpe kontinuierlich Edukt in einen Röhrenreaktor gefördert, der mit 5 g des Katalysators aus Beispiel 1 gefüllt und mit Hilfe eines Ölbades auf etwa 85 °C temperiert war. Bei einer Strömungsgeschwindigkeit von etwa 1 mL/min wurde ein Reaktionsprodukt erhalten welches rund 77 Gew.-% Limonen bei einer Selektivität von etwa 78 % aufwies.

## Patentansprüche

1. Verfahren zur Herstellung von Limonen, umfassend oder bestehend aus den folgenden Schritten:
(a) Bereitstellen von beta-Pinen oder einem beta-Pinen enthaltenden Ausgangsstoff;
(b) Versetzen des Ausgangsstoffes mit einer katalytisch wirksamen Menge eines MWW-Zeolithen vom Typ PSH-3;
(c) Erhitzen der Reaktionsmischung auf eine Temperatur im Bereich von 60 bis 100 °C und gegebenenfalls
(d) Abtrennen des Limonens oder einer an Limonen angereicherten Fraktion aus dem Sumpf.

2. Verfahren nach Anspruch 1, **dadurch gekennzeichnet, dass** man einen PSH-3 Zeolithen einsetzt, der zuvor kalziniert und/oder durch Säuren aktiviert worden ist.

3. Verfahren nach Anspruch 2, **dadurch gekennzeichnet, dass** man einen PSH-3 Zeolithen einsetzt, der zuvor über einen Zeitraum von 1 bis 10 Stunden bei einer Temperatur von 400 bis 1.000 °C kalziniert worden ist.

4. Verfahren nach Anspruch 2, **dadurch gekennzeichnet, dass** man einen PSH-3 Zeolithen einsetzt, der zuvor mit Salpetersäure aktiviert worden ist.

5. Verfahren nach mindestens einem der Ansprüche 1 bis 4, **dadurch gekennzeichnet, dass** man den Katalysator in einer Menge von 1 bis 5 Gew.-% bezogen auf die Menge an beta-Pinen einsetzt.

6. Verfahren nach mindestens einem der Ansprüche 1 bis 5, **dadurch gekennzeichnet, dass** man die Reaktion in Gegenwart von Lösungsmitteln durchführt.

7. Verfahren nach Anspruch 6, **dadurch gekennzeichnet, dass** man als Lösungsmittel einen Ester der Essigsäure mit einem aliphatischen C1-C4 Alkohol einsetzt.

8. Verfahren nach einem der Ansprüche 6 oder 7, **dadurch gekennzeichnet, dass** man das beta-Pinen und das Lösungsmittel im Gewichtsverhältnis von 10:1 bis 1:10 einsetzt.

9. Verfahren nach mindestens einem der Ansprüche 1 bis 8, **dadurch gekennzeichnet, dass** man die Reaktion über eine Dauer von 1 bis 10 Stunden durchführt.

10. Verfahren nach mindestens einem der Ansprüche 1 bis 9, **dadurch gekennzeichnet, dass** man den Katalysator nach der Reaktion gegebenenfalls ohne vorherige Aufarbeitung wiederverwendet.

11. Verfahren nach mindestens einem der Ansprüche 1 bis 10, **dadurch gekennzeichnet, dass** man das Verfahren batchweise oder kontinuierlich durchführt.

12. Verwendung eines Zeolithen vom PSH-3 Typ, gegebenenfalls nach vorheriger Kalzinierung und/oder Säureaktivierung, zur Umlagerung von beta-Pinen in Limonen.

## Claims

1. A process for the preparation of limonene comprising or consisting of the following steps:
(a) providing beta-pinene or a starting material containing beta-pinene;
(b) adding to the starting material a catalytically effective amount of a MWW zeolite of the PSH-3 type;
(c) heating the reaction mixture to a temperature in the range of 60 to 100 °C; and optionally
(d) separating the limonene or a fraction enriched in limonene from the sump.

2. The process according to claim 1, **characterized in that** a PSH-3 zeolite is used which has previously been calcined and/or activated by acids.

3. The process according to claim 2, **characterized in that** a PSH-3 zeolite is used which has previously been calcined over a period of 1 to 10 hours at a temperature of 400 to 1,000°C.

4. The process according to claim 2, **characterized in that** a PSH-3 zeolite which has previously been activated with nitric acid is used.

5. The process according to at least one of claims 1 to 4, **characterized in that** the catalyst is used in an amount of 1 to 5% by weight based on the amount of beta-pinene.

6. The process according to at least one of claims 1 to 5, **characterized in that** the reaction is carried out in the presence of solvents.

7. The process according to claim 6, **characterized in that** an ester of acetic acid with an aliphatic C1-C4 alcohol is used as solvent.

8. The process according to one of claims 6 or 7, **characterized in that** the beta-pinene and the solvent are used in a weight ratio of 10:1 to 1:10.

9. The process according to at least one of claims 1 to 8, **characterized in that** the reaction is carried out over a period of 1 to 10 hours.

10. The process according to at least one of claims 1 to 9, **characterized in that** the catalyst is reused after the reaction, if appropriate without prior work-up.

11. The process according to at least one of claims 1 to 10, **characterized in that** the process is carried out batchwise or continuously.

12. The use of a zeolite of the PSH-3 type, optionally after prior calcination and/or acid activation, for the rearrangement of beta-pinene into limonene.

## Revendications

1. Procédé de préparation de limonène, comprenant ou consistant en les étapes suivantes:
(a) fournir du bêta-pinène ou une matière première contenant du bêta-pinène ;
(b) ajouter à la matière première une quantité catalytiquement efficace d'une zéolithe MWW de type PSH-3 ;
(c) chauffage du mélange réactionnel à une température comprise entre 60 et 100 °C; et éventuellement
(d) la séparation du limonène ou d'une fraction enrichie en limonène du fond de la cuve.

2. Le procédé selon la revendication 1, **caractérisé en ce que** l'on met en oeuvre une zéolithe PSH-3 préalablement calcinée et/ou activée par des acides.

3. Le procédé selon la revendication 2, **caractérisé en ce que** l'on utilise une zéolithe PSH-3 préalablement calcinée pendant une durée de 1 à 10 heures à une température de 400 à 1000°C.

4. Le procédé selon la revendication 2, **caractérisé en ce que** l'on utilise une zéolithe PSH-3 préalablement activée par de l'acide nitrique.

5. Le procédé selon au moins l'une des revendications 1 à 4, **caractérisé en ce que** l'on met en oeuvre le catalyseur en une quantité de 1 à 5 % en poids par rapport à la quantité de bêta-pinène.

6. Le procédé selon au moins l'une des revendications 1 à 5, **caractérisé en ce que** l'on effectue la réaction en présence de solvants.

7. Le procédé selon la revendication 6, **caractérisé en ce que** l'on utilise comme solvant un ester de l'acide acétique avec un alcool aliphatique en C1-C4.

8. Le procédé selon l'une des revendications 6 ou 7, **caractérisé en ce que** l'on utilise le bêta-pinène et le solvant dans un rapport pondéral compris entre 10:1 et 1:10.

9. Le procédé selon au moins l'une des revendications 1 à 8, **caractérisé en ce que** l'on conduit la réaction sur une durée de 1 à 10 heures.

10. Le procédé selon au moins l'une des revendications 1 à 9, **caractérisé en ce que** l'on réutilise le catalyseur après la réaction, éventuellement sans retraitement préalable.

11. Le procédé selon au moins l'une des revendications 1 à 10, **caractérisé en ce que** l'on effectue le procédé en mode discontinu ou continu.

12. L'utilisation d'une zéolithe de type PSH-3, éventuellement après calcination et/ou activation acide préalable, pour le réarrangement du bêta-pinène en limonène.
